# EUROPEAN PATENT APPLICATION

(11) **EP 4 378 471 A1**
(43) Date of publication of application: **05.06.2024**
(21) Application number: 22848729.4
(22) Date of filing: 07.09.2022
(51) Int. Cl.: A61K 38/08, A61K 38/10, A61K 38/16, A61P 29/00, A61P 31/20, A61P 1/16, C07K 7/06, C07K 7/08, C07K 14/00

(54) **USE OF SE-DR AFFINITY PEPTIDE IN PREPARING DRUG FOR TREATING RHEUMATIC DISEASE**

(30) Priority: 30.07.2021 CN 202110873116
(71) Applicant: Hebei Fitness Biotechnology Co., Ltd., Shijiazhuang, Hebei 050035 (CN)
(72) Inventor: LIU, Chao, Shijiazhuang, Hebei 050035 (CN); HUANG, Lijing, Shijiazhuang, Hebei 050035 (CN); LI, Chenhui, Shijiazhuang, Hebei 050035 (CN); JIANG, Zhaohong, Shijiazhuang, Hebei 050035 (CN); WANG, Fei, Shijiazhuang, Hebei 050035 (CN); WANG, Hongquan, Shijiazhuang, Hebei 050035 (CN)
(74) Representative: Huang, Liwei
(86) International application number: PCT/CN2022/117495
(87) International publication number: WO 2023/006125

(57) **Abstract**

The present invention provides the use of SE-DR (an HLA-DR molecule containing the shared epitope) affinity peptides in the preparation of a medicament for the treatment of rheumatic diseases in the rheumatoid patients being tuberculosis-positive and/or complicated with hepatitis B. The invention solves the problem of limited choice of drugs for the patients with the rheumatic disease. The invention also provides a pharmaceutical composition comprising an SE-DR affinity peptide and a non-antigen-specific anti-rheumatic drug, and its use in the preparation of a medication for the treatment of various types of rheumatic diseases. The pharmaceutical composition can quickly delay the procession of the rheumatic disease, and can achieve sustained remission after the drug tapering or discontinuation, so as to completely control the rheumatic diseases.

## Description

### CROSS-REFERENCE TO RELATED APPLICATION

This application claims the benefit of Chinese application No. 202110873116.6, filed on July 30, 2021. The application No. 202110873116.6 is hereby incorporated herein by reference in its entirety.

### TECHNICAL FIELD

The invention belongs to the field of biopharmaceuticals, and particularly relates to the use of SE-DR affinity peptide in preparing a medication for treating rheumatic diseases in the rheumatoid patients being tuberculosis-positive and/or complicated with hepatitis B. In addition, the invention also relates to a pharmaceutical composition comprising the SE-DR affinity peptide and a non-antigen specific anti-rheumatic drug and its use in preparing drugs for treating various types of rheumatic diseases.

### BACKGROUND

Rheumatic disease is a group of extremely common clinical syndromes with arthralgia and fear of wind and cold as the main symptoms. Rheumatism is the abbreviation of rheumatic diseases, which generally refers to a large group of diseases affecting bones, joints, muscles and surrounding soft tissues, such as bursa, tendon, fascia, blood vessels, nerves and so on. Rheumatoid arthritis (RA) is one of the most common rheumatic diseases. It is a chronic, systemic autoimmune disease characterized by erosive synovitis, which can cause the destruction of joint cartilages and bones, resulting in joint deformity and eventually disability. The three-year disability rate of the patients without standard treatment is as high as 75%, which is one of the major reasons for the loss of labor force in young and middle-age population. The average life span of the patients is 3-10 years shorter than that of healthy people, and thus it is called "immortal cancer". The disease has caused serious social and economic problems. At the early stage of the disease, the patients still can work, however, 50% of the patients have lost their ability to work after 10 years, which will rise to 67% after 15 years. Rheumatoid arthritis is described as "5D" in foreign countries, that is, it can lead to Disease, Depression, Disability, Death and Debt. The prevalence of rheumatoid arthritis in the world is 0.5%-1%, and in China it is about 0.3%, and the number of patients is nearly 5 million. The peak age of onset in female patients in China is 35-44 years old, while that in male patients is 55-64 years old, and the incidence rate increases with age. It is more common in women, and the incidence ratio of women to men is 2:1-3:1.

The pathogenesis of rheumatic diseases is very complex. Under the influence of environmental factors or genetic factors, the pathogenic antigens in the susceptible population can activate the innate immune system, and the antigens are presented by antigen presenting cells (APC) to activate T cells. The activation of T cells requires co-stimulation by two independent signals. The first signal is the formation of a trimolecular complex of "human leukocyte antigen (HLA)-antigenic peptide-T cell receptor (TCR)", in which HLA and the antigenic peptide form a dimolecular complex first, and then associate with TCR to form a trimolecular complex; the second signal is the binding of CD28 on the surface of T cells to CD80/86 on the surface of APC cells. The activated T cells can further activate B cells, and these activated T cells and B cells enter the peripheral circulation and reach the lesion sites containing the autoantigens with structures similar to the pathogenic antigens. As the autoantigens serve as the pathogenic antigens, they can continuously activate T cells, B cells and the like, secrete a great amount of proinflammatory cytokines, chemokines and collagenases, and stimulate synovial hyperplasia, pannus formation, and persistent inflammation, eventually leading to bone destruction. T lymphocytes play an important role in the initiation, persistence and recurrence of rheumatoid arthritis, especially the differentiation of the activated T cells to the helper T cells, which plays an increasingly prominent role in the pathogenesis of rheumatoid arthritis and therapeutic drugs research. The changes of the Th17 cell and the Treg cell level have become the research focus in this field.

At present, the drugs for the treatment of rheumatic diseases (especially RA) can be divided into four generations based on the development time and the mechanism: the first generation is non-steroidal anti-inflammatory drugs (NSAID); the second generation is glucocorticoids (GC); the third generation is disease-modifying antirheumatic drugs (DMARD); the fourth generation is the biological agents represented by TNF-α inhibitors. The fourth-generation drugs for the treatment of rheumatic diseases include TNF-α inhibitors, IL-1 antagonists, IL-6 antagonists, JAK3 inhibitors, T cell inhibitors and B cell inhibitors, etc., currently accounting for up to 97% of the market in the United States, Japan and Europe. However, the commonly used drugs in clinical for the treatment of rheumatic diseases (especially RA) have a significant drawback in terms of safety and effectiveness.

At present, common NSAID, GC, DMARD and biological agents have some safety problems, which, to some extent, bring limitations to their clinical application. The half lethal dose of methotrexate, the first line drug recommended in clinical, in rats is 43mg/kg, and its main toxicities are myelosuppression and gastrointestinal tract toxicity. The preclinical toxicity of etanercept, one of the clinical second-line biological agents, is mainly manifested as latent tuberculosis infection, and its clinical toxicity is mainly to induce immune diseases, severe infections and increase the incidence of cancers; in the preclinical safety experiment for carcinogenesis of abatacept, about 50% of the mice in the treatment group died of lymphoma, and the incidence of breast cancer also increased. Clinical trials have demonstrated that abatacept can also aggravate infections and enhance the risk of cancers. Most biological agents (such as TNF inhibitors) are given a black box warning by FDA, indicating the risk of tumors and infections, so that patients are demanded to have a tuberculin test and hepatitis B related tests before administration. For the tuberculin-positive patients, biological agents should be applied only after the treatment for tuberculosis, and even if the tuberculin test is negative, active tuberculosis should be monitored during the application of biological agents; for the patients complicated with hepatitis B, preventive antiviral therapy should be given when HBcAb-positive patients start rituximab therapy, or when HBsAg-positive patients start any biological or targeted synthetic DMARD therapy; HBcAb-positive/HBsAg-negative patients should be closely monitored when initiating non-rituximab biological or targeted synthetic DMARD therapy. Therefore, for the tuberculosis-positive patients or the patients complicated with hepatitis B, there are problems of limited drug choice and increased risk of medication.

In addition, at present, the remission rate can only reach about 40% worldwide in the follow-up after two years of treatment using common clinical drugs with different regimens and different standards, and nearly half of the patients can not be effectively controled. In China, the clinical remission rate of RA patients is only 8.6%-25.2% when they are followed-up with different clinical criteria after treatment. A significant number of patients are unable to achieve clinical remission and disease control with current therapy.

The common reason for the safety problems of the existing clinical drugs that can alleviate rheumatic diseases (especially RA) can be attributed to "insufficient specificity to the target". NSAID, GC and DMARD have a wide range of immunosuppressive effects, but can not specifically inhibit the lymphocytes associated with the disease. Although the biological agents and novel small molecule drugs are targeted drugs for RA, most of them are aimed at the cytokines and kinases downstream in the pathogenesis of RA. While inhibiting the factors above, they also bring side effects, such as increasing the incidence of tuberculosis infection and tumors after inhibiting TNF-α. Abatacept is effective against the most upstream T-cell activation in the pathogenesis of RA. Although its safety is superior to that of TNF-α inhibitors and DMARD, its target is still against extensive T cell activation, rather than disease-specific T cells, which can cause immuno-deficiency, and further lead to severe infection and increased incidence of cancers during the treatment of the diseases. Therefore, the development of RA drugs targeting disease-specific targets is the main path to solve the safety problems of existing drugs.

SE-DR affinity peptide (such as FNS007) is an innovative drug independently developed in China, which directly acts on T lymphocytes, and can be considered as the fifth generation drug for the treatment of rheumatic diseases (especially RA). The SE-DR affinity peptide plays a role by competitively inhibiting the binding of rheumatic disease-related antigen peptide to the major histocompatibility complex molecules, and can interfere with the formation of the trimolecular complex "HLA-antigen peptide-T-lymphocyte receptor (TCR)", the first signal of the specific T cell activation mediated by rheumatic disease-related antigen. It can inhibit the activation and proliferation of the disease-specific autoreactive T cells, change the differentiation of T cells and the secretion of downstream cytokines, so as to achieve the purpose of treating rheumatic diseases (especially RA). "Antigen-specific therapy" is a major feature of the SE-DR affinity peptide mechanism. SE-DR affinity peptide is an altered peptide ligand of the main autoantigen epitope of RA, which acts on the specific stage of RA occurrence and development, is capable of inhibiting the binding of various RA-related antigen peptides with HLA molecules, and regulates the disordered immune microenvironment in the patients. By reducing the number of inflammatory cells and increasing the number of inflammation-suppressive cells, it is expected to restore immune tolerance and completely prevent the progress of the diseases by long-term correction. It can be said that the mechanism of SE-DR affinity peptide (for example, FNS007) in the treatment of rheumatic diseases represents the future development direction of anti-rheumatic drugs.

FNS007 is a novel class 1.1 drug developed by Hebei Fitness Biotechnology Co., Ltd., which has entered the clinical research stage and is now in phase I clinical research. The invention is an in-depth research on the new indication of SE-DR affinity peptides (especially FNS007) by the company aiming at the defects of the existing drugs for treating rheumatic diseases in terms of safety and effectiveness. Therefore, an object of the present invention is to provide the use of SE-DR affinity peptide in the preparation of a medicament for the treatment of rheumatic diseases in the rheumatoid patients being tuberculosis-positive and/or complicated with hepatitis B. Another object of the present invention is to provide a pharmaceutical composition comprising an SE-DR affinity peptide and a non-antigen-specific anti-rheumatic drug, and its use in the preparation of a medication for the treatment of various types of rheumatic diseases.

### SUMMARY

For the existing drugs for treating rheumatic diseases, how to make a balance between clinical safety and effectiveness is crucial. Increasing the dosage within a certain range can increase the efficacy of drugs and improve the remission rate, but at the same time can also increase the incidence of adverse reactions. Finding more disease-specific drugs for the treatment of rheumatic diseases and the related risks in the use of the drugs is the main path and new attempt to solve the problem of insufficient remission rate of the existing drugs and to meet the treatment demands of the patients with rheumatic diseases in China.

Antigen-specific therapy is a new idea to solve the clinical deficiencies of the above drugs for rheumatic diseases. Although some existing non-antigen specific drugs (such as TNF-α inhibitors) have now achieved some effectiveness, how to induce the body to restore tolerance to autoantigens is the key to the treatment of autoimmune diseases, and more specific targets also make it possible to reduce the toxicity of drugs, such as infection. Therefore, antigen-specific therapy, or its combination with non-antigen specific therapy, has become a new trend in the treatment of rheumatic diseases.

In order to achieve the purpose of the invention, the following technical proposal has been provided:
In the first aspect, the present invention provides the use of SE-DR affinity peptides, which refers to a peptide segment that is bound to an HLA DR molecule with shared epitope, in the preparation of a medicament for the treatment of rheumatic diseases in the rheumatoid patients being tuberculosis-positive and/or complicated with hepatitis B. Preferably, the shared epitope is a five animo acid motif with a QK/RRAA in the position of 70-74 of HLA-DRβ chain. The SE-DR affinity peptide plays a role by competitively inhibiting the binding of an antigen peptide related to rheumatic diseases with the HLA-DR molecule, but does not have a wide immunosuppressive effect and have no risk of inducing infection and tumors.

Alternatively, in the above uses, the core sequence of the SE-DR affinity peptide bound to SE-DR comprises core amino acids corresponding to P1 to P9, wherein the P1 site is an amino acid with a hydrophobic side chain, or a rare or unnatural amino acid with similar properties, and the P4 site is a nonpolar, polar and uncharged, polar and negatively charged amino acids, or a rare or unnatural amino acids with similar properties.

Preferably, the corresponding P1 site in the core sequence of the SE-DR affinity peptide bound to SE-DR is one selected from Tyr (Y), Phe (F), Trp (W), Leu (L), Ile (I), Met (M), Val (V) or Ala (A), and the P4 site is one selected from Met (M), Ala (A), Val (V), Ile (I), Leu (L), Asp (D), Glu (E), Gln (Q), Ser (S) or Cit.

More preferably, the corresponding P1 site in the core sequence of the SE-DR affinity peptide bound to SE-DR is one selected from Tyr (Y), Phe (F), or Trp (W), and the P4 site is one selected from Met (M), Leu (L), Asp (D), Glu (E), or Cit.

Alternatively, in the above uses, the corresponding P6 site in the core sequence of the SE-DR affinity peptide bound to SE-DR is an amino acid with a short side chain, or a rare or unnatural amino acid with similar properties, and the P9 site is an amino acid with a medium to short side chain, or a rare or unnatural amino acid with similar properties.

Preferably, the corresponding P6 site in the core sequence of the SE-DR affinity peptide bound to SE-DR is one selected from Ala (A), Gly (G), Ser (S), Thr (T) or Asn (N), and the P9 site is one selected from Ala (A), Gly (G), Leu (L) or Met (M).

Alternatively, in the above uses, the SE-DR affinity peptide comprises the following amino acid sequence: FXGEQGXXGE, or FXGEXAXXGE, wherein: X is selected from P, K, Q, A or G. Preferably, X is selected from A or G. More preferably, the SE-DR affinity peptide is FNS007 (FKGEQAGAGE).

Alternatively, in the above uses, the SE-DR affinity peptide comprises the following amino acid sequence: YXKQXTXXLA, wherein: X is selected from V, A, G, N, L or K.

Preferably, X is selected from A or G. More preferably, the amino acid sequence of the SE-DR affinity peptide is selected from the group consisting of PKYVKQNTLKLAT, PGYVKQGTLGLAT, YVKQNTLKLA, YVAQNTLKLA, YAKQATLKLA, or YAKQATLALA.

Alternatively, in the above uses, the SE-DR affinity peptide comprises the following amino acid sequence: IWYIXCFXCEXHXXL, wherein: X is selected from A, G, M, T, V, N, Q or S. Preferably, X is selected from A or G. More preferably, the amino acid sequence of the SE-DR affinity peptide is selected from IWYINCFGCETHAML, IWYIQCFGCETHAML, IWYISCFGCETHAML, IWYITCFGCETHAML, IWYINCFACETHAML or IWYINCFVCETHAML.

Alternatively, in the above uses, the SE-DR affinity peptide comprises the following amino acid sequence: RSFXLAXSXXGVG, wherein: X is selected from A, G, T, S or E.

Preferably, X is selected from A or G. More preferably, the amino acid sequence of the SE-DR affinity peptide is selected from RSFTLASSETGVG, RSFALASSETGVG, RSFTAASSETGVG, RSFTLDSSETGVG, RSFTLAASETGVG, RSFTLASSATGVG, RSFTLASSEAGVG or RSFTLDSSETGVG.

Alternatively, in the above uses, the SE-DR affinity peptide comprises the following amino acid sequence: SAVXLCitXSXXGVR, wherein: X is selected from A, G, R, S, V or P.

Preferably, the amino acid sequence of the SE-DR affinity peptide is SAVRLCitSSVPGVR, SAVELCitSSVPGVR, SAVDLCitSSVPGVR, SAVGLCitSSVPGVR, SAVRLCitFSVPGVR, SAVRLCitSSVEGVR, SAVRLCitSSVKGVR, SAVRLCitSSVWGVR, SAVRLCitWSVPGVR, SAVRLCitSSVRGVR, SAVRLCitKSVPGVR, SAVALCitSSVPGVR or SAVRLCitRSVPGVR. Alternatively, the SE-DR affinity peptide comprises the following amino acid sequence: GVYXTCitXSXXCitLCit, wherein: X is selected from A, G or V. Preferably, X is selected from A or G. More preferably, the amino acid sequence of the SE-DR affinity peptide is GVYATCitS SAVCitLCit.

Alternatively, the SE-DR affinity peptide comprises the following amino acid sequence: QDXNCitXNXXKNS, wherein: X is selected from A, G, F, I, K or L. Preferably, X is selected from A or G. More preferably, the amino acid sequence of the SE-DR affinity peptide is QDFTNCitANKLKNS.

Alternatively, the SE-DR affinity peptide comprises the following amino acid sequence: VVLLVATXGCitXRXXSAYQDK, wherein: X is selected from A, G, E, V or N. Preferably, X is selected from A or G. More preferably, the amino acid sequence of the SE-DR affinity peptide is VVLLVATEGCitVRVNSAYQDK.

Alternatively, in the above uses, the amino acid sequence of the SE-DR affinity peptide is selected from MGPKGRTVIIEQSWGSPKVTK, MGPKGRTVIIEQSLGSPKVTK, SIDLKDKKYKNIGAKLVQDVANNTNEEA, SIDLKDKKYKNIGAKL VQL VANNTNEEA, QYMCitADQAAGGLR, LTQCitGSVLR, WYNCitCHAAN, VETCitDGQVI or VCitLCitS SVES TCitGRSCitP APPP ACitGLT.

Alternatively, in the above uses, the rheumatic disease is one or more selected from rheumatoid arthritis, psoriasis, psoriatic arthritis, ankylosing spondylitis, undifferentiated spondyloarthropathy, systemic lupus erythematosus, systemic sclerosis or collagen disease.

In the second aspect, the present invention provides a pharmaceutical composition for the treatment of rheumatic diseases, which comprises an SE-DR affinity peptide and a non-antigen specific anti-rheumatic drug, as well as a pharmaceutically acceptable carrier. Preferably, the weight ratio of the SE-DR affinity peptide to the non-antigen specific anti-rheumatic drug ranges from 50:1 to 1:50, and more preferably, the weight ratio can be selected from 20:1-1:20, 15:1-1:15, 10:1-1:10, 5:1-1:5, 4:1-1:4, 3:1-1:3 or 2:1-1:2, and the specific dosage ratio is related to the type of the selected non-antigen-specific anti-rheumatic drug and can be determined by a clinician. The SE-DR affinity peptide refers to a peptide segment that is bound to an HLA-DR molecule with shared epitope, and preferably, the shared epitope is at the amino acid position 70-74 of the HLA-DR, and all expresses a QK/RRAA sequence. The SE-DR affinity peptide plays a role by competitively inhibiting the binding of an antigen peptide related to rheumatic diseases with the HLA-DR molecule, regulates the disordered immune microenvironment *in vivo*, and restores immunologic balance. The pharmaceutical composition rapidly delays the progression of rheumatic diseases and achieves sustained remission after drug tapering or discontinuation.

Alternatively, in the above pharmaceutical composition, the core sequence of the SE-DR affinity peptide bound to the SE-DR comprises core amino acids corresponding to P1 to P9, wherein the P1 site is an amino acid with a hydrophobic side chain, or a rare or unnatural amino acid with similar properties.

Preferably, the corresponding P1 site in the core sequence of the SE-DR affinity peptide bound to SE-DR is one selected from Tyr (Y), Phe (F), Trp (W), Leu (L), Ile (I), Met (M), Val (V) or Ala (A); and the P4 site is a non-polar, polar and uncharged, polar and negatively charged amino acid,, or a rare or unnatural amino acid with similar properties. Preferably, the corresponding P4 site in the core sequence of the SE-DR affinity peptide bound to SE-DR is one selected from Met (M), Ala (A), Val (V), Ile (I), Leu (L), Asp (D), Glu (E), Gln (Q) or Ser (S). The corresponding P6 site in the core sequence of the SE-DR affinity peptide bound to SE-DR is an amino acid containing a short side chain, or a rare or unnatural amino acid with similar properties, and the P9 site is an amino acid containing a medium to short side chain, or a rare or unnatural amino acid with similar properties. Preferably, the corresponding P6 site in the core sequence of the SE-DR affinity peptide bound to SE-DR is one selected from Ala (A), Gly (G), Ser (S), Thr (T) or Asn (N), and the P9 site is one selected from Ala (A), Gly (G), Leu (L) or Met (M).

The non-antigen specific anti-rheumatic drug is one or more selected from a drug targeting cytokines, a drug targeting B cells, a drug targeting T cells, a drug targeting JAK kinases, a traditional disease-modifying antirheumatic drug (DMARD), a non-steroidal anti-inflammatory drug (NSAID) or a glucocorticoids drug.

Preferably, the non-antigen specific anti-rheumatic drug is selected from one or more of a tumor necrosis factor (TNF) inhibitor, an IL-6 inhibitor, an IL-1 inhibitor, an IL-17 inhibitor, a RANKL inhibitor, a T-cell costimulatory signal inhibitor, a CD20 inhibitor, a CD22 inhibitor, a BLyS and APRIL inhibitor, a BAFF inhibitor, a JAK inhibitor, a BTK inhibitor, a Syk inhibitor, an IRAK4 inhibitor, a p38 inhibitor, or a small molecule immunosuppressant.

More preferably, the non-antigen specific anti-rheumatic disease drug is one or more selected from adalimumab, tocilizumab, anakinra, tofacitinib, abatacept, rituximab or their biosimilars or generic drugs thereof, methotrexate or leflunomide.

Alternatively, in the above pharmaceutical composition, the SE-DR affinity peptide comprises the follow amino acid sequence: FXGEQGXXGE, or FXGEXAXXGE, wherein: X is selected from K, Q, A, or G. Preferably, X is selected from A or G. More preferably, the SE-DR affinity peptide is FNS007 (FKGEQAGAGE).

Alternatively, the amino acid sequence of the SE-DR affinity peptide is selected from MGPKGRTVIIEQSWGSPKVTK, MGPKGRTVIIEQSLGSPKVTK , SIDLKDKKYKNIGAKLVQDVANNTNEEA, SIDLKDKKYKNIGAKL VQL VANNTNEEA, QYMCitADQAAGGLR, LTQCitGSVLR, WYNCitCHAAN, VETCitDGQVI or VCitLCitS SVES TCitGRSCitP APPP ACitGLT.

Alternatively, in the pharmaceutical composition described above, the pharmaceutically acceptable carrier includes a binder, a surfactant, a solubilizer, a stabilizer, a lubricant, a wetting agent and/or a diluent. The dosage form of the pharmaceutical composition is a capsule, a tablet, a pill, a liquid, a pulvis, a granule, a fine granule, a film coated tablet, a precipitant, a lozenge, a sublingual agent, a masticatory agent, a buccal agent, a paste, a syrup, a suspension, an elixir, an emulsion, a liniment, an ointment, a plaster, a poultice, a transdermal absorption formulation, a lotion, an inhalant, an aerosol, an injection or a suppository.

Alternatively, in the above pharmaceutical composition, the pharmaceutical composition is suitable for administration by a variety of routes, including oral, intravenous, subcutaneous, intramuscular, intracerebral, intranasal, pulmonary, intra-arterial, intra-articular, intradermal, intravitreal, intraosseous infusion, intraperitoneal, intrathecal, or transdermal administration.

Alternatively, in the above pharmaceutical composition, the rheumatic disease is one or more selected from rheumatoid arthritis, juvenile idiopathic arthritis, psoriasis, psoriatic arthritis, ankylosing spondylitis, undifferentiated spondyloarthropathy, systemic lupus erythematosus, systemic sclerosis, collagen disease, Crohn's disease, and ulcerative colitis.

Preferably, the patients suffering from the rheumatic diseases are tuberculosis-positive or tuberculosis-negative; and the patients suffering from the rheumatic diseases are or are not complicated with hepatitis B.

As compared with the prior art, the invention has the following beneficial effects:
(1) It is the first time to discover that the SE-DR affinity peptide (especially FNS007), an antigen-specific drug, has no significant effect on the immune system of the healthy people, healthy *cynomolgus* monkeys and healthy rats.
(2) It is the first time to discover that the SE-DR affinity peptide (especially FNS007) significantly reduced the incidence of tuberculosis in the type II collagen-induced arthritis model (CIA) rats latently infected with *Mycobacterium tuberculosis*, as compared with adalimumab.
(3) It is the first time to discover that the SE-DR affinity peptide (especially FNS007) significantly reduced the activation rate of hepatitis B in the type II collagen-induced arthritis model (CIA) mice infected with chronic hepatitis B, as compared with abatacept.
(4) It is the first time to discover that the combination of an antigen-specific drug (especially, FNS007, APL20) and a non-antigen-specific drug (such as adalimumab, abatacept, or methotrexate) can rapidly delay the progression of diseases in the CIA rats, and is safer than adalimumab, abatacept, or methotrexate used alone, and the remission sustained especially after the tapering or discontinuation of the drugs.
(5) The antigen-specific drug (such as FNS007, APL20) can be used in combination with other non-antigen-specific drugs to completely control of rheumatoid arthritis.

### BRIEF DESCRIPTION OF THE DRAWINGS

Fig. 1: Inflammation scores at various time points during administration in the CIA rat model in the experiment shown in Example 9.
Fig. 2: Inflammation scores at various time points during drug discontinuation in the CIA rat model in the experiment shown in Example 9.

### DETAILED DESCRIPTION

The invention is further described below with reference to specific Examples. It should be understood that the specific Examples described herein are merely illustrative of the invention and are not intended to limit the scope of the invention.

If no specific technology or conditions are specified in the Examples, the technology or conditions described in the literatures in this field or in the product specification shall be followed. If the manufacturers of the reagents or the instruments used are not indicated, these reagents or instruments are all conventional products that can be purchased through regular channels.

The experimental methods in the following Examples are all conventional methods, unless otherwise specified. The test materials used in the following Examples are all commercially available, unless otherwise specified.

In the invention, the polypeptide used is synthesized by Hebei Fitness Biotechnology Co., Ltd. using solid-phase synthesis and purified using high performance liquid chromatography. The analysis result of the polypeptide by mass spectrometry has shown that the sequence of the polypeptide is correct, and the purity is more than 95%.

### Part I: In vitro experiment results of the SE-DR affinity peptides

The SE-DR molecule is directly related to the pathogenesis of RA. It can present RA-related autoantigens *in vivo*, such as type II collagen (CII), heat shock protein 60 (Hsp60), cartilage glycoprotein (HCgp-39) and so on, and further activate autoreactive T cells, leading to the occurrence of rheumatoid arthritis. An important strategy for the treatment of RA is to use competitive inhibition to block the activation of autoreactive T cells by autoantigen peptides. At present, the main idea is to modify the autoantigen peptides, so that it can not only inhibit the binding of autoantigens, but also not activate autoreactive T cells, which requires that the altered peptide ligands have a certain affinity for SE-DR, but also show reduced T-cell activation. Based on the above principles, we have designed and synthesized a series of altered peptide ligands, and screened for their affinity to SE-DR and for the activation ability on the peripheral blood T cells in RA patients. The specific steps and results are shown in Example 1 and Example 2.

### Example 1: Determination of polypeptide affinity

Set-up of reaction system: in a 96-well plate, 200 µL reaction system was established for each well, containing 500 nM HLA-DR1, 25 nM fluorescently-labeled polypeptide and the test polypeptide, which was 5-fold diluted starting from 200 µM for 5 gradients. As the quality control well, the last well did not contain the test polypeptide. The quality control well used a fluorescently-labeled polypeptide that did not contain the MHCII molecule to measure the free polypeptide signal. The reaction system was at pH 7.4. Before use, protease inhibitor cocktail was added , and incubated at 37 °C for 30 min. The fluorescence polarization values were read. The mean value of the free labeled polypeptides was set as FP_free, and the value of the well without the competitive peptide was set as FP_no_comp. The relative binding rate of other wells was calculated according to the formula = (FP_sample - FP_free)/(FP_no_comp - FP_free). A curve was plotted using the relative binding rate and the concentration of the test polypeptide, and the IC₅₀ value was calculated by curve fitting.

By comprehensively considering the amino acid properties of the SE-DR binding and non-binding polypeptides, it can be identified that P1, P4, P6, and P9 are the key amino acids affecting the affinity between the polypeptide and SE-DR. Among others, P1 is an amino acid with a hydrophobic side chain, or a rare or unnatural amino acid with similar properties; P4 site is a non-polarity, polarity and uncharged, polarity and negatively-charged amino acid, or a rare or unnatural amino acid with similar properties; P6 site is an amino acid with a short side chain, or a rare or unnatural amino acid with similar properties; and the P9 site is an amino acid with a medium to short side chain, or a rare or unnatural amino acid with similar properties.

**Table 1. IC₅₀ values of the SE-DR binding polypeptides calculated by curve fitting**

| **Polypeptide No.** | | **Polypeptide Sequence** | | | | | | | | | | **IC50 (nM)** |
|---|---|---|---|---|---|---|---|---|---|---|---|---|
| | | **P1** | **P2** | **P3** | **P4** | **P5** | **P6** | **P7** | **P8** | **P9** | | |
| APL1 | | F | K | G | E | Q | G | G | A | G | E | 12468 |
| APL2 | | F | K | G | E | Q | A | G | A | G | E | 3369 |
| APL3 | PG | Y | V | K | Q | G | T | L | G | L | AT | 3347 |
| APL4 | | Y | V | K | Q | N | T | L | K | L | A | 8674 |
| APL5 | | Y | V | A | Q | N | T | L | K | L | A | 6683 |
| APL6 | | Y | A | K | Q | A | T | L | K | L | A | 6672 |
| APL7 | | Y | A | K | Q | A | T | L | A | L | A | 4634 |
| APL8 | IWYIQC | F | G | C | E | T | H | A | M | L | | 5469 |
| APL9 | IWYISC | F | G | C | E | T | H | A | M | L | | 5087 |
| APL10 | IWYITC | F | G | C | E | T | H | A | M | L | | 5865 |
| APL11 | IWYINC | F | A | C | E | T | H | A | M | L | | 3977 |
| APL12 | IWYINC | F | V | C | E | T | H | A | M | L | | 5201 |
| APL13 | RS | F | A | L | A | S | S | E | T | G | VG | 5023 |
| APL14 | RS | F | T | A | A | S | S | E | T | G | VG | 6541 |
| APL15 | RS | F | T | L | D | S | S | E | T | G | VG | 8966 |
| APL16 | RS | F | T | L | A | A | S | E | T | G | VG | 5546 |
| APL17 | RS | F | T | L | A | S | S | A | T | G | VG | 3529 |
| APL18 | RS | F | T | L | A | S | S | E | A | G | VG | 4876 |
| APL19 | RS | F | T | L | D | G | S | E | T | G | VG | 7753 |
| APL20 | SIDLKDKKYKNIGAK | L | V | Q | L | V | A | N | N | T | NEEA | 3876 |
| APL21 | MGPKGRT | V | I | I | E | Q | S | L | G | S | PKVTK | 4148 |
| APL22 | SA | V | E | L | Cit | S | S | V | P | G | VR | 7753 |
| APL23 | SA | V | D | L | Cit | S | S | V | P | G | VR | 6642 |
| APL24 | SA | V | A | L | Cit | S | S | V | P | G | VR | 5899 |
| APL25 | SA | V | G | L | Cit | S | S | V | P | G | VR | 5768 |
| APL26 | SA | V | R | L | Cit | R | S | V | P | G | VR | 6942 |
| APL27 | SA | V | R | L | Cit | F | S | V | P | G | VR | 8977 |
| APL28 | SA | V | R | L | Cit | S | S | V | E | G | VR | 10654 |
| APL29 | SA | V | R | L | Cit | S | S | V | K | G | VR | 18892 |
| APL30 | SA | V | R | L | Cit | S | S | V | W | G | VR | 16554 |
| APL31 | SA | V | R | L | Cit | W | S | V | P | G | VR | 27853 |
| APL32 | SA | V | R | L | Cit | S | S | V | R | G | VR | 26997 |
| APL33 | SA | V | R | L | Cit | K | S | V | P | G | VR | 20581 |
| APL34 | GV | Y | A | T | Cit | S | S | A | A | Cit | LCit | 4937 |
| APL35 | QD | F | T | N | Cit | A | N | K | L | K | NS | 10858 |
| APL36 | VVLLUATEGCitVRVASAYQDK | | | | | | | | | | | 5320 |
| APL37 | VCitLCitSSVESTCitGRSCitPAPPPACitGLT | | | | | | | | | | | 5537 |

| | | | | | | | | | | | | |
|---|---|---|---|---|---|---|---|---|---|---|---|---|
| (Note: P1-P9 in the table indicates the binding sites of the polypeptide to HLA-DR. Due to the particularity of polypeptide APL36 and APL37, the binding sites are not indicated.) | | | | | | | | | | | | |

**Table 2. IC₅₀ values of the non-SE-DR binding polypeptides calculated by curve fitting**

| **Polypeptide No.** | | **Polypeptide Sequence** | | | | | | | | | | **IC₅₀ (nM)** |
|---|---|---|---|---|---|---|---|---|---|---|---|---|
| | | **P1** | **P2** | **P3** | **P4** | **P5** | **P6** | **P7** | **P8** | **P9** | | |
| APL38 | | A | K | G | E | Q | A | G | A | G | E | >100000 |
| APL39 | | G | K | G | E | Q | A | G | A | G | E | >100000 |
| APL40 | | F | K | G | A | Q | A | G | A | G | E | 69654 |
| APL41 | | F | K | G | R | Q | A | G | A | G | E | 88392 |
| APL42 | | A | K | G | E | Q | A | G | A | W | E | >100000 |
| APL43 | | A | K | G | E | Q | A | G | A | Y | E | >100000 |
| APL44 | | A | V | K | Q | N | T | L | K | L | A | 77923 |
| APL45 | | G | V | K | Q | N | T | L | K | L | A | 67963 |
| APL46 | | A | V | K | Q | N | A | L | K | L | A | >100000 |
| APL47 | | A | V | K | Q | N | T | L | K | Y | A | >100000 |
| APL48 | | A | V | K | Q | N | T | L | K | W | A | >100000 |
| APL49 | | A | V | K | R | N | T | L | K | W | A | >100000 |
| APL50 | | A | V | K | H | N | T | L | K | W | A | >100000 |
| APL51 | | A | V | K | R | N | A | L | K | W | A | >100000 |
| APL52 | | A | V | K | H | N | A | L | K | W | A | >100000 |
| APL53 | IWYINC | A | G | C | E | T | A | A | M | L | | >100000 |
| APL54 | IWYINC | G | G | C | E | T | A | A | M | L | | >100000 |
| APL55 | IWYINC | F | G | C | R | T | A | A | M | L | | 98792 |
| APL56 | IWYINC | F | G | C | H | T | A | A | M | L | | 69163 |
| APL57 | IWYINC | A | G | C | E | T | A | A | M | W | | >100000 |
| APL58 | IWYINC | A | G | C | E | T | A | A | M | Y | | >100000 |
| APL59 | RS | A | T | L | A | S | A | E | T | G | VG | 88911 |
| APL60 | RS | G | T | L | A | S | A | E | T | G | VG | 61663 |
| APL61 | RS | A | T | L | R | S | A | E | T | G | VG | >100000 |
| APL62 | RS | G | T | L | R | S | A | E | T | G | VG | >100000 |

| | | | | | | | | | | | | |
|---|---|---|---|---|---|---|---|---|---|---|---|---|
| (Note: P1-P9 in the table indicates the binding sites of the polypeptide to HLA-DR) | | | | | | | | | | | | |

### Example 2: PBMC stimulation test

3 mL ficoll was added to a 15 mL centrifuge tube, and 4 mL blood sample was then applied on the surface of ficoll. The tube was centrifuged at 400 g at 18-20 °C for 30-40 min. The upper layer of plasma was aspirated with a Pasteur pipette, and the PBMC layer (approximately 2 mL) was aspirated, and added to a 14 mL centrifuge tube previously filled with at least 3 volumes (6 mL) of PBS. The mixture was gently resuspended. The tube was centrifuged at 60-100 g for 10 min at 18-20 °C. After centrifugation, the supernatant was removed with a Pasteur pipette, and the resulting cell mass was resuspended in 6-8 mL PBS and centrifuged at 18-20 °C for 10 min at 60-100 g to remove the supernatant. The cells were resuspended in an appropriate amount of 1640 complete medium and diluted to 1×10⁶ cell/ml after cell counting. The diluted cell suspension was added to a 96-well plate. The positive control, allosteric peptide, was added to 100 µl cell culture to a final concentration of 10 µg/ml. The mixture was replenished to 200 µL with 1640 complete medium. After 5 days of culture, the supernatant was aspirated, centrifuged at 3000 rpm for 5 minutes, and the level of IL-6 in the supernatant was detected by ELISA.

**Table 3. IL-6 levels in the supernatant detected by ELISA**

| **Polypeptide No.** | **IL-6 (ng/ml)** |
|---|---|
| APL1 | 18.9 |
| APL2 | 2.3 |
| APL5 | 8.7 |
| APL6 | 6.5 |
| APL7 | 3.7 |
| APL9 | 2.8 |
| APL 10 | 4.9 |
| APL15 | 8.7 |
| APL16 | 6.8 |
| APL17 | 5.4 |
| APL19 | 15.8 |
| APL20 | 2.7 |
| APL21 | 4.2 |
| APL24 | 6.6 |
| APL26 | 7 |
| APL34 | 5.2 |
| APL35 | 20.8 |
| APL36 | 6.8 |
| APL37 | 4.5 |

Through Example 1 and 2, pharmacodynamic studies were conducted on some of the SE-DR affinity peptides at animal level.

### Part II: Pharmacodynamic results of the SE-DR affinity peptides

### 1. The SE-DR affinity peptides were capable of significantly inhibiting the progression of diseases in CIA rats and promotes the differentiation of peripheral blood T-cells to Th2 and Treg cells in CIA rats (Example 3)

Collagen-induced arthritis (CIA) model was established by intradermal injection of the emulsion formulated using type II bovine collagen (CII) together with incomplete Freund's adjuvant (IFA) into the tail head of a female Lewis rat. After the model establishment, the morphological changes of rats' four paws were examined every day. When the inflammation score was 1, the diseased rats were randomly divided into the model group, the SE-DR affinity peptide group (administration of FNS007 (APL2), APL7, APL9, APL20, ALP21, and ALP37, respectively), the autoantigen peptide group (i.e., Type II collagen CII263-272), and the irrelevant peptide group. Except for the model group, the dosage of polypeptide was all set as 0.2 mmol. A control group was also set up, in which the drug was injected into the tail vein once every other day for 15 consecutive days. The area under the curve (AUC) of the inflammation score was used for the efficacy analysis from the day the rats were enrolled, once every other day, until the end of administration. In addition, blood samples were collected from the orbital angular vein at 144 h after the end of the administration, after which the proportions of helper T-cells (Th1, Th2, Th17) and T regulatory cells (Treg) in the peripheral blood mononuclear cells were measured by flow cytometry.

The results showed that the effects of the SE-DR affinity peptides (FNS007 (APL2), APL7, APL9, APL20, ALP21 and ALP37) on the changes in the proportion of T-cell subtypes in the peripheral blood of the rats after multiple administration for 144 h were shown in Table 4. As compared with the control group, the proportions of Th1 and Th17 cells and the ratio of Th1/Th2 cells in the model group increased significantly (p<0.05, p<0.01), while the proportions of Th2 and Treg cells decreased significantly (p<0.05, p<0.01). As compared with the model group, the proportions of Th1 and Th17 cells in the SE-DR affinity peptide group were significantly reduced (p<0.05, p<0.01), while the proportions of Th2 and Treg cells was significantly enhanced (p<0.05, p<0.01). The results were shown in Table 4. In conclusion, the SE-DR affinity peptides promote the differentiation of the peripheral blood T cells from proinflammatory Th1 and Th2 cells to anti-inflammatory Th2 and Treg cells in the CIA rats.

**Table 4. Changes in the proportions of the T-cell subtypes in peripheral blood (x̅ ±s)**

| **Group** | **Dosage** | **Inflammation score and area under the time curve (AUC)** | **Th1 (%)** | **Th2 (%)** | **Th17 (%)** | **Treg (%)** |
|---|---|---|---|---|---|---|
| Control Group | -- | 0.00±0.00 | 0.15±0.04 | 4.41±0.88 | 0.69±0.09 | 5.96±0.66 |
| Model Group | -- | 109.42±40.11^{##} | 0.39±0.17^{# #} | 3.53±0.52^{# #} | 1.08±0.08^{# #} | 4.15±0.69^{# #} |
| FNS007 (APL2) | 0.2mmol | 52.37±33.69** | 0.16±0.09* * | 4.53±0.50* | 0.87±0.09* * | 5.52±0.68* * |
| APL7 | 0.2mmol | 60.48±37.31** | 0.17±0.10* * | 4.38±0.81* | 0.92±0.08 | 5.22±0.77* |
| APL9 | 0.2mmol | 57.39±39.33** | 0.20±0.05* | 4.27±0.53 | 0.95±0.12* | 5.35±0.84* |
| APL20 | 0.2mmol | 55.31±40.23** | 0.18±0.07* * | 4.35±0.62* | 0.88±0.07* * | 5.46±0.81* * |
| APL21 | 0.2mmol | 59.44±38.29** | 0.19±0.08* | 4.23±0.47 | 0.98±0.10 | 5.14±0.90* |
| ALP37 | 0.2mmol | 63.55±36.28** | 0.18±0.06* * | 4.18±0.77 | 0.97±0.15 | 5.27±0.73* |
| Prototype Peptide Group | 0.2mmol | 83.24±42.51 | 0.24±0.05 | 4.34±0.90 | 1.12±0.16 | 3.94±0.78 |
| Irrelevant Peptide Group | 0.2mmol | 91.27±36.88 | 0.29±0.08 | 3.78±0.94 | 1.24±0.38 | 4.69±0.71 |

| | | | | | | |
|---|---|---|---|---|---|---|
| Note: as compared with the control group, ^{#}*P*<0.05, ^{##}*P*<0.01; as compared with the model group, **P*<0.05, ***P*<0.01. | | | | | | |

### 2. Experiment results of the SE-DR affinity peptides in the tuberculosis-positive RA subjects

### 2.1 The SE-DR affinity peptides do not interfere with normal immune functions

Because the SE-DR affinity peptide only inhibits the activation of the T cells related to autoantigen recognition and has no influence on the normal immune system of the body protecting from foreign infection, the SE-DR affinity peptide has no risk of infection and tumors, and can be used for tuberculosis-positive rheumatoid patients. Therefore, the invention can provide a novel therapeutic drug for tuberculosis-positive RA patient, and solve the problem that the antirheumatic biological drug can not be used in this type of RA patient.

Studies have shown that the SE-DR affinity peptides will not significantly change the immune system of healthy people, healthy monkeys and healthy rats, suggesting that the SE-DR affinity peptides do not interfere with the normal immune functions of the body, do not reduce the defense capacity of the body, and have no risk of infection and tumors. See in particular Example 4 to Example 6 below.

### Example 4

SD rats were used to investigate the effects of the SE-DR affinity peptides on the immune indicators of the rats after continuous administration for one month. The SE-DR affinity peptides were divided into 6 dose groups (FNS007 (APL2), APL7, APL9, APL20, ALP21, ALP37), and a control control group was also set up, with 10 male and 10 female animals in each group, administered once a day for 4 weeks. At the end of administration, analysis was conducted on the serum anti-FNS007 antibodies, different types of serum immunoglobulins, the serum complement, the peripheral blood T lymphocyte and the serum cytokines.

The results showed that after administration of the SE-DR affinity peptide, as compared with the control group, there was no significant change in all immune indicators, suggesting that the SE-DR affinity peptide had no effect on the immune system of healthy rats. Table 5 shows the results of the effect of the SE-DR affinity peptide on the immune cells in healthy rats.

**Table 5. The effect of the SE-DR affinity peptide on the immune cells in rats.**

| **Group** | **CD3%** | **CD8%** | **CD4%** | **IL-2 pg/mL** | **TNF-α pg/mL** | **IL-4 pg/mL** | **IFN-γ pg/mL** |
|---|---|---|---|---|---|---|---|
| Control Group | 39.18±9. 71 | 17.83±2.8 9 | 20.11±7.76 | 3.63±3.58 | 5.10±1.85 | 2.17±1.55 | 2.67±0.40 |
| FNS007 (APL2) | 37.4±10. 52 | 17.29±7.0 9 | 19.77±6.05 | 3.69±3.03 | 5.13±1.83 | 1.96±1.26 | 2.47±0.36 |
| APL7 | 36.69±17 | 18.66±8.6 1 | 18.29±9.08 | 3.06±0.97 | 5.42±1.23 | 2.55±1.30 | 2.33±0.29 |
| APL9 | 36.08±12 .9 | 17.10±8.8 2 | 20.45±8.45 | 3.03±1.05 | 4.79±1.08 | 1.95±0.95 | 2.35±0.27 |
| APL20 | 36.96±9. 81 | 17.99±8.2 3 | 18.96±5.98 | 3.55±2.75 | 5.09±1.88 | 2.03±1.01 | 2.58±0.29 |
| APL21 | 38.16±8. 98 | 19.06±5.4 | 18.72±5.85 | 3.36±1.45 | 5.25±1.28 | 2.32±1.37 | 2.64±0.37 |
| ALP37 | 38.29±11 .34 | 18.33±6.1 5 | 19.25±7.84 | 3.28±2.59 | 4.96±2.01 | 1.77±0.85 | 2.44±0.31 |

### Example 5

*Cynomolgus* monkeys were used to investigate the effects of the SE-DR affinity peptide on the immune indicators of *cynomolgus* monkeys after continuous administration for one month. The administration scheme of the SE-DR affinity peptide was divided into 6 dosage groups (FNS007 (APL2), APL7, APL9, APL20, ALP21, ALP37), and a control group was also set up, with 3 male and 3 female animals in each group, administered once a day for 4 consecutive weeks. At the end of administration, the immune indicators, including the serum anti-FNS007 antibodies, the immunoglobulins of different serotypes, the serum complement, the peripheral blood T lymphocyte and the serum cytokines, were observed.

The results showed that after administration, no significant changes were observed for all immune indicators as compared to the control group, indicating that the SE-DR affinity peptide had no effect on the immune system of healthy monkeys. Table 6 shows the effect of the SE-DR affinity peptide on the immune cells of healthy monkeys.

**Table 6. The effect of the SE-DR affinity peptide on the immune cells of monkeys.**

| **Group** | **CD3%** | **CD8%** | **CD4%** | **IL-2 pg/mL** | **TNF-α pg/mL** | **IL-4 pg/mL** | **IFN-γ pg/mL** |
|---|---|---|---|---|---|---|---|
| Control Group | 66.73± 8.31 | 22.00± 11.5 | 26.86± 2.33 | 4.46± 0.45 | 2.37± 0.35 | 2.07± 0.11 | 5.13± 0.24 |
| FNS007 (APL2) | 63.37± 16.35 | 21.74± 4.37 | 27.33± 6.66 | 4.62± 0.86 | 2.42± 0.51 | 2.12± 0.42 | 5.09± 0.52 |
| APL7 | 61.83± 11.98 | 23.72± 6.35 | 26.23± 7.31 | 4.24± 0.34 | 2.44± 0.22 | 2.04± 0.19 | 5.27± 0.59 |
| APL9 | 66.91± 11.93 | 28.67± 14.78 | 28.67± 14.78 | 4.60± 0.55 | 2.38± 0.24 | 2.24± 0.12 | 5.01± 0.69 |
| APL20 | 62.96± 9.77 | 22.97± 7.09 | 27.82± 8.05 | 4.55± 0.71 | 2.45± 0.38 | 2.16± 0.36 | 5.18± 0.33 |
| APL21 | 66.16± 4.34 | 23.43± 8.46 | 23.43± 8.46 | 4.18± 0.41 | 2.16± 0.14 | 1.90± 0.15 | 5.03± 0.22 |
| ALP37 | 64.55± 17.31 | 27.61± 9.99 | 28.84± 9.35 | 4.46± 0.44 | 2.51± 0.41 | 2.18± 0.29 | 5.11± 0.54 |

### Example 6

The effects of a single-dose administration of the SE-DR affinity peptide on human immune indicators were investigated in healthy volunteers. The SE-DR affinity peptide used was FNS007, and the administration scheme was divided into three dosage groups (5,10 and 20 mg/case), and a placebo control group was also set up. After administration, the immune indicators, including the immunoglobulins of different serotypes, the peripheral blood T lymphocyte and the serum cytokines etc., were observed.

The results showed that after administration, no significant changes were observed for all immune indicators, as compared to the placebo group and the baseline levels before administration, indicating that the SE-DR affinity peptide had no effect on the immune system of healthy people. Table 7 shows the effect of the SE-DR affinity peptide on the immune cells of healthy people.

**Table 7. The effect of the SE-DR affinity peptide on the immune cells of healthy people.**

| **Group** | **CD3%** | **CD8%** | **CD4%** | **TGFβ ng/mL** | **TNF-α pg/mL** | **IL-10 pg/mL** | **IFN-γ pg/mL** |
|---|---|---|---|---|---|---|---|
| Placebo Group | 48.55± 6.72 | 44.73± 9.34 | 37.16± 5.36 | 18.24± 2.75 | 1.95± 0.48 | 0.95± 0.57 | 7.56± 7.21 |
| Low Dosage Group | 47.95± 5.34 | 43.38± 7.21 | 37.11± 5.01 | 19.73± 2.56 | 1.91± 0.28 | 0.89± 0.75 | 7.45± 6.16 |
| Medium Dosage Group | 47.66± 4.35 | 46.95± 6.27 | 36.25± 4.89 | 18.10± 3.24 | 1.96± 0.35 | 0.91± 0.48 | 7.89± 5.93 |
| High Dosage Group | 49.87± 7.01 | 44.15± 6.88 | 38.01± 6.11 | 19.12± 3.11 | 1.95± 0.29 | 1.01± 0.72 | 8.14± 5.15 |

### 2.2 The SE-DR affinity peptide has no risk of inducing tuberculosis infection

In order to further compare the differences between the SE-DR affinity peptide and the biological agents in inducing infection, the CIA model rat latently infected with tuberculosis was used in this study to compare the incidences of tuberculosis in the rats during the treatment of arthritis with adalimumab and the SE-DR affinity peptide.

The results showed that both adalimumab and the SE-DR affinity peptide had a good therapeutic effect on the CIA rats, but adalimumab tended to induce the recurrence of tuberculosis during the treatment, with the incidence of tuberculosis as high as 60%. While no recurrence of tuberculosis was observed in the model group and the SE-DR affinity peptide group. As demonstrated above, the SE-DR affinity peptide has a good therapeutic effect on rheumatoid arthritis, does not increase the incidence of tuberculosis infection and is expected to be safe for tuberculosis-positive RA patients. See Example 7 for details.

### Example 7

Establishment of the latently infected rat model: female Lewis rats were infected intravenously with 5×10³ CFU (colony forming unit) Erdman strain. The amount of *Mycobacterium tuberculosis* (MTB) in lung and spleen tissues of rats could reach more than 1×10⁴ at 4 weeks after infection. At this time, the anti-tuberculosis drugs isoniazid (0. 1 g/L) and rifampicin (15 g/L) were added to the drinking water for 4 weeks. The cultures of MTB from the lung and spleen of rats were negative after 2 weeks of drug discontinuation, suggesting successful establishment of the latent tuberculosis infection model.

The CIA model of latent tuberculosis infection was established using the female Lewis rats with latent tuberculosis infection described above. Methods: The rats with latent tuberculosis infection were immunized by intradermal injection of emulsion formulated with bovine C II and IFA (containing 100 µg collagen) at the tail head. After 7 days, the rats were boosted once with the same method. The morphological changes of the four paws of the rats were examined every day. The rats were randomly divided into model groups for treatment at the inflammation score of 1. The diseased rats were randomly divided into the model group, the SE-DR affinity peptide group (FNS007 (APL2), APL7, APL9, APL20, ALP21, ALP37, 0.2 mmol) and 1 mg/kg adalimumab group, with 10 rats in each group. Another 8 rats with latent infection were not subjected to model establishment and set as the control group. PBS was administrated to both the model group and the control group. The rats in each group were given the corresponding drugs by tail intravenous injection at the volume of 5 ml/kg, once every other day, for 21 consecutive days. The rats were sacrificed on day 22 after administration. During the administration, the inflammation scores of the four paws of the rats in each group were evaluated, and the MTB culture of lungs and spleens of rats was detected at the time of sacrifice.

After model establishment, as compared with the control group, the inflammation score of the model rats was significantly increased. In order to reflect the influence of the drug on the index during the whole course of disease and to evaluate the overall effect of the drug, the area under the curve (AUC) of the inflammation score was statistically analyzed. The results showed that as compared with the model group, the SE-DR affinity peptide group and the adalimumab group could significantly reduce the inflammation score of the diseased rats (P<0.01). At the time of sacrifice, the cultures of *Mycobacterium tuberculosis* from the lungs and spleens of the rats in the control group, the model group and the SE-DR affinity peptide group were all negative, but in the adalimumab group, the cultures of *Mycobacterium tuberculosis* from the lungs and spleens were positive in 6/10 rats. In addition, the animals in the adalimumab group had obvious toxicity symptoms such as piloerection and reduced activity during the administration, while the rats were in good condition after the administration of the SE-DR affinity peptide. The above results suggest that the SE-DR affinity peptide does not increase the risk of recurrence of tuberculosis in rats, and its safety is superior to adalimumab. The results are shown in Table 8.

**Table 8. The therapeutic effect of the SE-DR affinity peptide on CIA rats with latent tuberculosis infection and the recurrence of tuberculosis**

| **Group** | **Inflammation score and area under the time curve (AUC)** | **Culture of MTB from lungs and spleens of rats at sacrifice** | **Tuberculosis recurrence rate** | **Observation on the daily condition of rats** |
|---|---|---|---|---|
| Control Group (n=8) | 0.00±0.00 | Negative | 0 | No abnormality |
| Model Group (n=10) | 100.13±39.27^{##} | Negative | 0 | No abnormality |
| FNS007 (APL2) (n=10) | 48.03±31.91** | Negative | 0 | No abnormality |
| APL7 (n=10) | 55.93±29.87** | Negative | 0 | No abnormality |
| APL9 (n=10) | 54.49±33.12** | Negative | 0 | No abnormality |
| APL20 (n=10) | 49.62±35.87** | Negative | 0 | No abnormality |
| APL21 (n=10) | 53.06±37.25** | Negative | 0 | No abnormality |
| APL37 (n=10) | 53.19±29.18** | Negative | 0 | No abnormality |
| Adalimumab Group (n=10) | 50.42±27.13** | 6/10 Positive | 60% | Piloerection, prostration, reduced activity, etc. |

| | | | | |
|---|---|---|---|---|
| Note: As compared with the control group, ^{#}*P*<0.05, ^{##}*P*<0.01; as compared with the model group, **P*<0.05, ***P*<0.01. | | | | |

### 2.3 The SE-DR affinity peptide has no risk of inducing hepatitis B (HBV) infection

In order to further compare the differences between the SE-DR affinity peptide and the biological agents in inducing infection, the CIA mouse model with chronic hepatitis B infection was used in this study to compare the activation of hepatitis B in mice during the treatment of arthritis with abatacept and the SE-DR affinity peptide.

The results showed that both abatacept and the SE-DR affinity peptide had a good therapeutic effect on CIA mice, but abatacept was tended to activate hepatitis B during the course of treatment, with the ALT level increased to >100 IU/ml in 50% mice. While there was no significant difference in the ALT level among the SE-DR affinity peptide group, the model group and the control group. It is suggested that the SE-DR affinity peptide will not increase the activation rate of hepatitis B while treating rheumatoid arthritis, and it is expected to be safely used in RA patients infected with hepatitis B. See Example 8 for details.

### Example 8

Establishment of chronic hepatitis B model in mice: DBA/1 mice were injected with Raav8-1. 3HBV (1×10¹² vg/mL) via tail vein to establish HBV chronic hepatitis B model.

DBA/1 mice infected with chronic hepatitis B were used to establish CIA model with chronic hepatitis B infection. Methods: Two days after the mice were injected with Raav8-1.3HBV (1×10¹² vg/mL) via tail vein, the emulsion of bovine C II and CFA (containing 100 µg collagen) was intradermally injected into the tail head of the mice for immunization. After 21 days, the mice were boosted once by intraperitoneal injection of the emulsion (containing 100 µg collagen) prepared by bovine C II and IFA. Thereafter, the morphological changes of the four paws of the mice were examined every day, and the mice were randomly assigned to the treatment group at inflammation score of 1. The diseased mice were randomly divided into the model group, the SE-DR affinity peptide group (treated with FNS007 (APL2), APL7, APL9, APL20, ALP21, and ALP37, respectively, at 50 µmol) and Abatacept group (100 µg), with 12 mice in each group. Another 10 mice with latent hepatitis B infection were not subjected to model establishment and set as the control group. PBS was administrated to both the model group and the control group. The mice in each group were given the corresponding drugs by tail intravenous injection at the volume of 10 ml/kg, once every other day, for 27 consecutive days. The mice were sacrificed on day 28 after administration. The inflammation scores of the four paws of the mice in each group were evaluated during the administration. The serological indicators for virus injection and liver function of the mice were detected on the day of booster immunization (22 days after tail vein injection) and at the time of sacrifice.

After model establishment in the mice, as compared with the control group, the inflammation scores of the model mice were significantly increased. In order to reflect the influence of the drug on the index during the whole course of disease and to evaluate the overall effect of the drug, the area under the curve (AUC) of the inflammation score was statistically analyzed. The results showed that as compared with the model group, the SE-DR affinity peptide group and the abatacept group could significantly reduce the inflammation score of the diseased mice (P<0.01). In the evaluation of hepatitis B virus, HBsAg, HBeAg and HBV DNA were detected in all mice at the time of booster immunization, that is, 22 days after tail intravenous injection, and the average levels of ALT and AST were 45 and 65 U/L, respectively. It is suggested that the chronic infection model of hepatitis B has been successfully established. At the time of sacrifice, the serological indicators for liver function of the mice in the control group, the model group and the SE-DR affinity peptide group were similar to those at the time of booster immunization, but the serological indicators for liver function of the mice in the abatacept group were significantly increased, with the ALT level increased to >100 IU/ml in 50% of the mice, suggesting that the risk of hepatitis B activation in the mice was greatly increased. In addition, the animals had obvious toxicity symptoms such as piloerection and reduced activity during the administration of abatacept, while the mice were in good condition after the administration of the SE-DR affinity peptide. These results suggest that the SE-DR affinity peptide does not increase the risk of hepatitis B activation in the mice, and its safety is better than that of abatacept. See Table 9 for the results of each index at the time of final sampling.

**Table 9. The therapeutic effect of the SE-DR affinity peptide on the CIA mice infected with chronic hepatitis B and the activation of hepatitis B**

| **Group** | **Inflammation score and area under the time curve (AUC)** | **ALT level (U/L)** | **Hepatitis B activation rate (Ratio of ALT > 100U/L)** | **Observation on the daily condition of mice** |
|---|---|---|---|---|
| Control Group (n=10) | 0.00±0.00 | 44.15±16.29 | 0 | No abnormality |
| Model Group (n=12) | 167.21±60.95^{##} | 46.23±20.11 | 0 | No abnormality |
| FNS007 (APL2) (n=12) | 89.86±46.14** | 45.88±19.74 | 0 | No abnormality |
| APL7 (n=12) | 96.19±48.25** | 48.53±20.09 | 0 | No abnormality |
| APL9 (n=12) | 97.63±50.13** | 47.55±18.78 | 0 | No abnormality |
| APL20 (n=12) | 92.34±53.29** | 46.28±17.99 | 0 | No abnormality |
| APL21 (n=12) | 95.71±51.88** | 48.29±21.24 | 0 | No abnormality |
| APL37 (n=12) | 96.45±49.73** | 47.37±21.13 | 0 | No abnormality |
| Abatacept Group (n=12) | 88.29±48.40** | 103.59±32.84 | 50% | Piloerection, prostration, reduced activity, etc. |

| | | | | |
|---|---|---|---|---|
| Note: As compared with the control group, ^{#}*P*<0.05, ^{##}*P*<0.01; as compared with the model group, **P*<0.05, ***P*<0.01. | | | | |

### 3. Experiment results of the combined dosage regimen for RA subjects

The antigen-specific therapy combined with non-antigen-specific therapy is the future direction of rheumatoid treatment. On one hand, non-antigen-specific therapy can facilitate antigen-specific therapy to control clinical symptoms (such as inflammation and so on) as soon as possible, on the other hand, through the immunomodulatory effect of the antigen-specific drugs, immune tolerance can be rebuilt and diseases can be completely controlled.

In order to verify this theory, the CIA model was used in this study to investigate the combination of the SE-DR affinity peptide (antigen-specific therapeutic drug) with adalimumab/abatacept/methotrexate (non-antigen-specific therapeutic drug), and to compare the differences between the combination therapy with the SE-DR affinity peptide, adalimumab, abatacept or methotrexate alone. The results showed that the SE-DR affinity peptide combined with adalimumab, the SE-DR affinity peptide combined with abatacept, and the SE-DR affinity peptide combined with methotrexate could rapidly control the inflammatory response in rats as compared with the SE-DR affinity peptide alone.

As compared with administration of adalimumab, abatacept, or methotrexate alone, the combination group was more effective and safer. The SE-DR affinity peptide group and the combination group maintained the therapeutic effect even after the drug discontinuation, while the adalimumab, abatacept, or methotrexate group showed enhanced inflammatory scores after the drug discontinuation. It is suggested that the combination of the SE-DR affinity peptide and a non-antigen-specific therapeutic drug (such as adalimumab/abatacept/methotrexate) can greatly improve the clinical remission rate, and can sustain remission even after drug discontinuation, so as to achieve drug tapering or discontinuation. See Example 9, Example 10 and Example 11 for details.

### Example 9

Female Lewis rats were used to establish CIA model. Methods: The rats were immunized by intradermal injection with the emulsion prepared by bovine C II and IFA at the tail head. After 7 days, the rats were boosted once with the same method. Thereafter, the morphological changes of the four paws of the rats were examined every day. The rats were randomly assigned to the treatment group at the inflammation score of 1. The diseased rats were randomly divided into three groups, i.e., the model group, the SE-DR affinity peptide group (FNS007, 0.2 mmol), the adalimumab group (1 mg/kg), and the combination therapy group (FNS007 0.2 mmol + adalimumab 1 mg/kg), with 10 rats in each group. Another 8 female Lewis rats were not subjected to model establishment and set as the control group. PBS was administrated to both the model group and the control group. Corresponding drugs were administrated to the rats in each group by tail intravenous injection at a volume of 5 mL/kg, once every other day, for 21 consecutive days, followed by a recovery period of 21 days with drug discontinuation. The inflammation scores of the four paws of the rats in each group were evaluated during the administration (once every other day), and the area under the curve (AUC) of the inflammation score was calculated during the administration. During drug discontinuation, the inflammation scores of the four paws of the rats in each group were continuously evaluated (once every other day), and the rats were sacrificed on day 22 after drug discontinuation. HE staining was performed on the ankle joint for tissue injury evaluation. The specific scoring criteria were as follows: 0 = normal synovial tissue; 1 = synovial hyperplasia and inflammatory cell infiltration; 2 = pannus formation and cartilage erosion; 3 = destruction of most of the cartilage with subchondral bone erosion; 4 = loss of joint integrity, ankylosis.

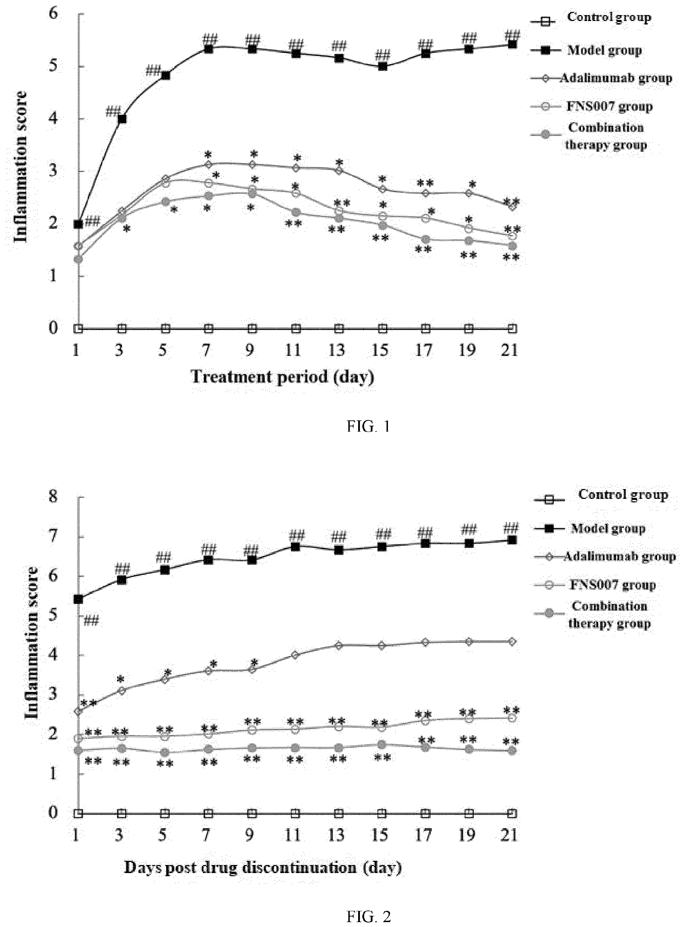

The results showed that in the CIA rat model, as compared with the group of FNS007 alone, the combination therapy group of FNS007 and adalimumab could rapidly control the inflammatory response of rats, which could be demonstrated by lower inflammatory score in the combination therapy group relative to that in the group of FNS007 alone in first week during administration, with the inflammatory score AUC decreased from 14.26 to 11.93. There was a significant difference between these two groups (P<0.05). The inflammation score AUC of the combination therapy group was significantly reduced during the whole administration period, with the inflammatory score AUC reduced from 46. 17 to 35. 88. There was a significant difference between these two groups (P<0.05). In addition, as compared with adalimumab, the efficacy of the combination therapy group was more significant (inflammation score AUC decreased from 54. 51 to 35. 88, with statistical difference, P<0.05), and the incidence of infection in rats was reduced, and the symptoms of piloerection and epistaxis in rats were significantly improved. At the same time, the administration was stopped at day 21, followed by a period of 21 days for observation. During drug discontinuation, the FNS007 group and the combination therapy group could maintain the therapeutic effect, while the adalimumab group could not, which was reflected by the significantly increased inflammation score as compared with that at the time of drug discontinuation (P<0.01). The results are shown in Table 10 and Fig. 1 and 2.

**Table 10. The therapeutic effects of FNS007 in combination with adalimumab in CIA rats**

| **Group** | **AUC, the first week during administration** | **AUC, during administration (day 1-21)** | **AUC, during drug discontinuation (day 22-42)** | **Pathological score during 21 days of drug discontinuation** | **Observation on the daily state of rats** | **Incidence of infection in rats** |
|---|---|---|---|---|---|---|
| Control Group (n=8) | 0.00±0.00 | 0.00±0.00 | 0.00±0.00 | 0.00±0.00 | No abnormality | 0 |
| Model Group (n=10) | 25.00±5.26^{##} | 98.42±46.08^{##} | 129.83±54.11^{##} | 3.33±0.65^{##} | No abnormality | 0 |
| FNS007 Group (n=10) | 14.26±3.87** | 46.17±38.62** | 42.86±31.21** | 1.93±0.58** | No abnormality | 0 |
| Adalimumab Group (n=10) | 13.93±4.81** | 54.51±22.72** | 96.85±26.51 | 2.69±0.80 | Piloerection, epistaxis, prostration, reduced activity, etc. | 10% |
| Combination Therapy Group (n=10) | 11.93±3.55* | 35.88±33.91** | 32.91±29.57** | 1.68±0.67** | Slight piloerection | 0 |

| | | | | | | |
|---|---|---|---|---|---|---|
| Note: as compared with the control group, ^{#}*P*<0.05, ^{##}*P*<0.01; as compared with the model group, **P*<0.05, ***P*<0.01. | | | | | | |

### Example 10

Male DBA/1 mice were used to establish CIA model. Methods: Mice were immunized by intradermally injecting an emulsion of bovine C II and CFA (containing 100 µg collagen) at the tail head. After 21 days, the mice were boosted once by intraperitoneally injecting an emulsion of bovine C II and IFA (containing 100 µg collagen). Thereafter, the morphological changes of the four paws of the mice were examined every day, and the mice were randomly assigned to the treatment group at inflammation score of 1. The diseased mice were randomly divided into three groups: the model group, the SE-DR affinity peptide group (FNS007, 0.2 mmol), the abatacept group (5 mg/kg), and the combination therapy group (FNS007 0.2 mmol + abatacept 5 mg/kg), with 12 mice in each group. Another 10 female DBA/1 mice were not subjected to model establishment and set as the control group. PBS was administrated to both the model group and the control group. Corresponding drugs were administrated to the mice in each group by tail vein injection at the volume of 10 mL/kg, once every other day, for 28 consecutive days, followed by a recovery period of 28 days. The inflammation score of the four paws of the mice in each group was evaluated during the administration (once every other day), and the area under the curve (AUC) of the inflammation score was calculated during the administration. During drug discontinuation, the inflammation scores of the four paws of the rats in each group were continuously evaluated (once every other day), and the rats were sacrificed on day 28 after drug discontinuation. HE staining was performed on the ankle joint for tissue injury evaluation. The specific scoring criteria were as follows: 0 = normal synovial tissue; 1 = synovial hyperplasia and inflammatory cell infiltration; 2 = pannus formation and cartilage erosion; 3 = destruction of most of the cartilage with subchondral bone erosion; 4 = loss of joint integrity, ankylosis.

The results showed that in the CIA mouse model, as compared with the group of FNS007 alone, the combination therapy group of FNS007 and abatacept could rapidly control the inflammatory response of the mouse, which could be demonstrated by a lower inflammatory score in the combination therapy group relative to that in the group of FNS007 alone in the first 10 days during administration, with the inflammatory score AUC decreased from 25.64 to 18.11. There was a significant difference between the two groups (P < 0.05). The inflammation score AUC of the combination therapy group was significantly reduced from 90.23 to 75.87 during the whole administration period, and there was a significant difference between the two groups (P < 0.05). In addition, as compared with the abatacept group, the efficacy of the combination therapy group was more significant (inflammation score AUC decreased from 89.16 to 75.87, with statistical difference, P<0.05), and the incidence of infection in the mice was reduced, and the symptoms of piloerection and epistaxis were significantly improved. At the same time, the administration was stopped at day 28, followed by a period of 28 days for observation. During drug discontinuation, the FNS007 group and the combination therapy group could maintain the therapeutic effect, while the abatacept group could not, which was reflected by the significantly increased inflammation score as compared with that at the time of drug discontinuation (P<0.01). The results are shown in table 11.

**Table 11: The therapeutic effects of FNS007 in combination with abatacept in CIA mice**

| **Group** | **AUC, the first week during administration** | **AUC, during administration (day 1-28)** | **AUC, during drug discontinuation (day 29-56)** | **Pathological score during 28 days of drug discontinuation** | **Observation on the daily state of mice** | **Incidence of infection in mice** |
|---|---|---|---|---|---|---|
| Control Group (n=10) | 0.00±0.00 | 0.00±0.00 | 0.00±0.00 | 0.00±0.00 | No abnormality | 0 |
| Model Group (n=12) | 48.50±18.47^{##} | 170.55±62.34^{##} | 210.54±78.44^{##} | 3.92±0.28^{##} | No abnormality | 0 |
| FNS007 Group (n=12) | 25.64±9.05** | 90.23±45.28** | 87.55±40.08** | 2.05±0.87** | No abnormality | 0 |
| Abatacept Group (n=12) | 23.33±9.17** | 89.16±51.44** | 143.55±77.82 | 2.89±0.80 | Piloerection, epistaxis, prostration, reduced activity, etc. | 16.7% |
| Combination Therapy Group (n=12) | 18.11±7.99* | 75.87±39.68** | 76.47±40.01** | 1.61±0.96** | Slight piloerection | 0 |

| | | | | | | |
|---|---|---|---|---|---|---|
| Note: as compared with the control group, ^{#}*P*<0.05, ^{##}*P*<0.01; as compared with the model group, **P*<0.05, ***P*<0.01. | | | | | | |

### Example 11

Female Lewis rats were used to establish CIA model. Methods: the rats were immunized by intradermal injecting the emulsion prepared by bovine C II and IFA at the tail head. After 7 days, the rats were boosted once with the same method. Thereafter, the morphological changes of the four paws of the rats were examined every day, and the rats were randomly assigned to the treatment group at the inflammation score of 1. The diseased rats were randomly divided into three groups, i.e., the model group, the SE-DR affinity peptide group (APL20, 0.2 mmol), the methotrexate group (0.5 mg/kg), and the combination therapy group (APL20 0.2 mmol + methotrexate 0.5 mg/kg), with 10 rats in each group. Another 8 female Lewis rats were not subjected to model establishment and set as the control group. PBS was administrated to both the model group and the control group. Corresponding drugs were administrated to the rats in each group by tail vein injection at the volume of 5 mL/kg, in which methotrexate was administrated twice a week, and other drugs were administrated once every other day, for 21 consecutive days, followed by a recovery period of 21 days. The inflammation scores of the four paws of the rats in each group were evaluated during the administration (once every other day), and the area under the curve (AUC) of the inflammation score was calculated during the administration. During drug discontinuation, the inflammation scores of the four paws of the rats in each group were continuously evaluated (once every other day), and the rats were sacrificed on day 22 after drug discontinuation. HE staining was performed on the ankle joint for tissue injury evaluation. The specific scoring criteria were as follows: 0 = normal synovial tissue; 1 = synovial hyperplasia and inflammatory cell infiltration; 2 = pannus formation and cartilage erosion; 3 = destruction of most of the cartilage with subchondral bone erosion; 4 = loss of joint integrity, ankylosis.

The results showed that in the CIA rat model, as compared with the group of APL20 alone, the combination therapy group of APL20 and methotrexate could rapidly control the inflammatory response of the rat, which could be demonstrated by a lower inflammatory score in the combination therapy group relative to that in the group of FNS007 alone in the first weeks during administration, with the inflammatory score AUC decreased from 14.93 to 12.06. There was a significant difference between the two groups (P < 0.05). The inflammation score AUC of the combination therapy group was significantly reduced from 48.69 to 37.59 during the whole administration period, and there was a significant difference between the two groups (P < 0.05). In addition, as compared with methotrexate, the efficacy of the combination therapy group was more significant (inflammation score AUC decreased from 67.69 to 37.59, with statistical difference, P<0.05), and the symptoms of piloerection and epistaxis in rats were significantly improved, and the mortality of animals was reduced. At the same time, the administration was stopped at day 21, followed by a period of 21 days for observation. During drug discontinuation, the APL20 group and the combination therapy group could maintain the therapeutic effect, while the methotrexate group could not, which was reflected by the significantly increased inflammation score as compared with that at the time of drug discontinuation (P<0.01). The results are shown in Table 12.

**Table 12: The therapeutic effect of APL20 in combination with methotrexate in CIA rats**

| **Group** | **AUC, the first week during administrat -ion** | **AUC, during administration (day 1-21)** | **AUC, during drug discontinuation (day 22-42)** | **Pathological score during 21 days of drug discontinuation** | **Observation on the daily state of mice** | **Incidence of infection in mice** |
|---|---|---|---|---|---|---|
| Control Group (n=8) | 0.00±0.00 | 0.00±0.00 | 0.00±0.00 | 0.00±0.00 | No abnormality | 0 |
| Model Group (n=10) | 26.18±4.96^{# #} | 100.27±45.78^{##} | 141.47±55.44^{##} | 3.57±0.49^{##} | No abnormality | 0 |
| APL20 Group (n=10) | 14.93±4.11* * | 48.69±40.06** | 50.11±41.27** | 2.05±0.34** | No abnormality | 0 |
| Methotrexate Group (n=10) | 16.58±5.23* * | 67.69±22.72* | 100.13±50.66 | 2.87±0.79 | Piloerection, epistaxis, prostration, reduced activity, etc. | 20% |
| Combination Therapy Group (n=10) | 12.06±3.79* * | 37.59±32.16** | 39.08±35.66** | 1.96±0.55** | Slight piloerection | 0 |

| | | | | | | |
|---|---|---|---|---|---|---|
| Note: as compared with the control group, ^{#}*P*<0.05, ^{##}*P*<0.01; as compared with the model group, **P*<0.05, ***P*<0.01. | | | | | | |

As demonstrated above, the combination of the SE-DR affinity peptide group and the non-antigen-specific therapeutic drug (such as adalimumab/abatacept/methotrexate) can greatly improve the clinical remission rate, and can sustain remission even after drug discontinuation, so as to achieve drug tapering or discontinuation. Therefore, the present invention can provide a novel pharmaceutical composition that can significantly improve the clinical remission rate and is expected to achieve drug tapering or discontinuation.

It will be apparent to those skilled in the art that various modifications and variations can be made in the present invention without departing from the spirit and scope of the invention. Thus, the present invention is intended to include such modifications and variations provided they come within the scope of the appended claims and their equivalents.

## Claims

1. Use of SE-DR affinity peptides in the preparation of a medicament for treating rheumatic diseases of rheumatoid patients being tuberculosis-positive and/or complicated with hepatitis B, which is **characterized in that**: the SE-DR affinity peptide refers to a peptide that is bound to an HLA-DR molecule with shared epitope, and preferably, the shared epitope is a five animo acid motif with a QK/RRAA in the position of 70-74 of HLA-DR β chain, and the SE-DR affinity peptide plays a role by competitively inhibiting the binding of an disease-related autoantigen peptide with the HLA-DR molecule, but does not have a wide immunosuppressive effect, and has no risk of inducing infection and tumors.

2. The use according to claim 1, which is **characterized in that**: the core sequence of the SE-DR affinity peptide bound to SE-DR comprises core amino acids corresponding to P1 to P9, wherein the P1 site is an amino acid with a hydrophobic side chain, or a rare or unnatural amino acid with similar properties, and the P4 site is a non-polar, polar and uncharged, polar and negatively charged amino acid, or a rare or unnatural amino acid with similar properties,
preferably, a corresponding P1 site in the core sequence of the SE-DR affinity peptide bound to SE-DR is one selected fromTyr (Y), Phe (F), Trp (W), Leu (L), Ile (I), Met (M), Val (V) or Ala (A), and the P4 site is one selected fromMet (M), Ala (A), Val (V), Ile (I), Leu (L), Asp (D), Glu (E), Gln (Q), Ser (S) or Cit,
and more preferably, the corresponding P1 site in the core sequence of the SE-DR affinity peptide bound to SE-DR is one selected from Tyr (Y), Phe (F) or Trp (W), and the P4 site is one selected from Met (M), Leu (L), Asp (D), Glu (E) or Cit.

3. The use according to claim 2, which is **characterized in that**: the corresponding P6 site in the core sequence of the SE-DR affinity peptide bound to SE-DR is an amino acid with a short side chain, or a rare or unnatural amino acids with similar properties, and the P9 site is an amino acids with a medium to short side chain, or a rare or unnatural amino acids with similar properties,
preferably, the corresponding P6 site in the core sequence of the SE-DR affinity peptide bound to SE-DR is one selected from Ala (A), Gly (G), Ser (S), Thr (T) or Asn (N), and the P9 site is one selected from Ala (A), Gly (G), Leu (L) or Met (M).

4. The use according to claim 1, which is **characterized in that**: the SE-DR affinity peptide comprises the following amino acid sequence: FXGEQGXXGE or FXGEXAXXGE, wherein: X is selected from P, K, Q, A or G ,
preferably, X is selected from A or G,
and more preferably, the SE-DR affinity peptide is FNS007 (FKGEQAGAGE).

5. The use according to claim 1, which is **characterized in that**: the SE-DR affinity peptide comprises the following amino acid sequence: YXKQXTXXLA, wherein: X is selected from V, A, G, N, L or K,
preferably, X is selected from A or G,
and more preferably, the amino acid sequence of the SE-DR affinity peptide is selected from PKYVKQNTLKLAT, PGYVKQGTLGLAT, YVKQNTLKLA, YVAQNTLKLA, YAKQATLKLA or YAKQATLALA.

6. The use according to claim 1, which is **characterized in that**: the SE-DR affinity peptide comprises the following amino acid sequence: IWYIXCFXCEXHXXL, wherein: X is selected from A, G, M, T, V, N, Q or S,
preferably, X is selected from A or G,
and more preferably, the amino acid sequence of the SE-DR affinity peptide is selected from IWYINCFGCETHAML, IWYIQCFGCETHAML, IWYISCFGCETHAML, IWYITCFGCETHAML, IWYINCFACETHAML or IWYINCFVCETHAML.

7. The use according to claim 1, which is **characterized in that**: the SE-DR affinity peptide comprises the following amino acid sequence: RSFXLAXSXXGVG, wherein: X is selected from A, G, T, S or E,
preferably, X is selected from A or G,
and more preferably, the amino acid sequence of the SE-DR affinity peptide is selected from RSFTLASSETGVG, RSFALASSETGVG, RSFTAASSETGVG, RSFTLDSSETGVG, RSFTLAASETGVG, RSFTLASSATGVG, RSFTLASSEAGVG or RSFTLDSSETGVG.

8. The use according to claim 1, which is **characterized in that**:
the SE-DR affinity peptide comprises the following amino acid sequence: SAVXLCitXSXXGVR, wherein: X is selected from A, G, R, S, V or P, and preferably, the amino acid sequence of the SE-DR affinity peptide is SAVRLCitSSVPGVR, SAVELCitSSVPGVR, SAVDLCitSSVPGVR, SAVGLCitSSVPGVR, SAVRLCitFSVPGVR, SAVRLCitSSVEGVR, SAVRLCitSSVKGVR, SAVRLCitSSVWGVR, SAVRLCitWSVPGVR, SAVRLCitSSVRGVR, SAVRLCitKSVPGVR, SAVALCitSSVPGVR or SAVRLCitRSVPGVR;
alternatively, the SE-DR affinity peptide comprises the following amino acid sequence: GVYXTCitXSXXCitLCit, wherein: X is selected from A, G or V, and preferably, X is selected from A or G, and more preferably, the amino acid sequence of the SE-DR affinity peptide is GVYATCitS SAVCitLCit;
alternatively, the SE-DR affinity peptide comprises the following amino acid sequence: QDXNCitXNXXKNS, wherein: X is selected from A, G, F, I, K or L, and preferably, X is selected from A or G, and more preferably, the amino acid sequence of the SE-DR affinity peptide is QDFTNCitANKLKNS;
alternatively, the SE-DR affinity peptide comprises the following amino acid sequence: VVLLVATXGCitXRXXSAYQDK, wherein: X is selected from A, G, E, V or N, and preferably, X is selected from A or G, and more preferably, the amino acid sequence of the SE-DR affinity peptide is VVLLVATEGCitVRVNSAYQDK.

9. The use according to claim 1, which is **characterized in that**: the amino acid sequence of the SE-DR affinity peptide is selected from: MGPKGRTVIIEQSWGSPKVTK, MGPKGRTVIIEQSLGSPKVTK, SIDLKDKKYKNIGAKLVQDVANNTNEEA, SIDLKDKKYKNIGAKL VQL VANNTNEEA, QYMCitADQAAGGLR, LTQCitGSVLR, WYNCitCHAAN, VETCitDGQVI or VCitLCitSSVESTCitGRSCitPAPPPACitGLT.

10. The use according to any one of claims 1-9, which is **characterized in that**: the rheumatic disease is one or more selected from rheumatoid arthritis, psoriasis, psoriatic arthritis, ankylosing spondylitis, undifferentiated spondyloarthropathy, systemic lupus erythematosus, systemic sclerosis or collagen disease.

11. A pharmaceutical composition for the treatment of rheumatic diseases, which is **characterized in that**: The pharmaceutical composition comprises an SE-DR affinity peptide, a non-antigen specific anti-rheumatic drug, and a pharmaceutically acceptable carrier, wherein the SE-DR affinity peptide refers to a peptide segment that is bound to an HLA-DR molecule with shared epitope, and preferably, the shared epitope is a five animo acid motif with a QK/RRAA in the position of 70-74 of HLA-DR β chain, and the SE-DR affinity peptide plays a role by competitively inhibiting the binding of an disease-related autoantigen peptide with the HLA-DR molecule, improves the disordered immune microenvironment in the body of a patient, restores the immune balance, and the pharmaceutical composition rapidly inhibits the progression of rheumatic disease and achieves sustained remission after drug discontinuation.

12. The pharmaceutical composition according to claim 11, wherein the core sequence of the SE-DR affinity peptide bound to SE-DR comprises core amino acids corresponding to P1 to P9, wherein the P1 site is an amino acid with a hydrophobic side chain, or a rare or unnatural amino acid with similar properties, and preferably, the corresponding P1 site in the core sequence of the SE-DR affinity peptide bound to SE-DR is one selected from Tyr (Y), Phe (F), Trp (W), Leu (L), Ile (I), Met (M), Val (V) or Ala (A); the P4 site is a non-polar, polar and uncharged, polar and negatively charged amino acid, or a rare or unnatural amino acid with similar properties, and preferably, the corresponding P4 site in the core sequence of the SE-DR affinity peptide bound to SE-DR is one selected from Met (M), Ala (A), Val (V), Ile (I), Leu (L), Asp (D), Glu (E), Gln (Q) or Ser (S); The corresponding P6 site in the core sequence of the SE-DR affinity peptide bound to SE-DR is an amino acid containing a short side chain, or a rare or unnatural amino acids with similar properties, and the P9 site is an amino acid containing a medium to short side chain, or a rare or unnatural amino acids with similar properties, and preferably, the corresponding P6 site in the core sequence of the SE-DR affinity peptide bound to SE-DR is one selected from Ala (A), Gly (G), Ser (S), Thr (T) or Asn (N), and the P9 site is one selected from Ala (A), Gly (G), Leu (L) or Met (M);
the non-antigen specific anti-rheumatic drug is one or more selected from a drug targeting cytokines, a drug targeting B cells, a drug targeting T cells, a drug targeting kinases, a traditional disease-modifying antirheumatic drug (DMARD), a non-steroidal anti-inflammatory drug (NSAID), or a glucocorticoids, preferably, the non-antigen specific anti-rheumatic drug is selected from that group consisting of a tumor necrosis factor (TNF) inhibitor, an IL-6 inhibitor, an IL-1 inhibitor, an IL-17 inhibitor, a RANKL inhibitor, a T-cell costimulatory signal inhibitor, a CD20 inhibitor, a CD22 inhibitor, a BLyS and APRIL inhibitor, a BAFF inhibitor, A JAK inhibitor, a BTK inhibitor, a Syk inhibitor, an IRAK4 inhibitor, a p38 inhibitor, or a small molecule immunosuppressant,
and more preferably, the drug for the non-antigen specific anti-rheumatic disease is one or more selected from adalimumab, tocilizumab, anakinra, tofacitinib, abatacept, rituximab or their biosimilars or generic drugs thereof, methotrexate or leflunomide.

13. The pharmaceutical composition according to claim 11 or claim 12, which is **characterized in that**: the SE-DR affinity peptide comprises the following amino acid sequence: FXGEQGXXGE, or FXGEXAXXGE, wherein: X is selected from K, Q, A or G, and preferably, X is selected from A or G, and more preferably, the SE-DR affinity peptide is FNS007 (FKGEQAGAGE), or the amino acid sequence of the SE-DR affinity peptide is selected from MGPKGRTVIIEQSWGSPKVTK, MGPKGRTVIIEQSLGSPKVTK , SIDLKDKKYKNIGAKLVQDVANNTNEEA, SIDLKDKKYKNIGAKL VQL VANNTNEEA, QYMCitADQAAGGLR, LTQCitGSVLR, WYNCitCHAAN, VETCitDGQVI or VCitLCitS SVES TCitGRSCitP APPP ACitGLT.

14. The pharmaceutical composition according to any one of claims 11-13, which is **characterized in that**: the pharmaceutically acceptable carrier includes a binder, a surfactant, a solubilizer, a stabilizer, a lubricant, a wetting agent and/or a diluent, and the dosage form of the pharmaceutical composition is a capsule, a tablet, a pill, a liquid, a pulvis, a granule, a fine granule, a film coated tablet, a precipitant, a lozenge, a sublingual agent, a masticatory agent, a buccal agent, a paste, a syrup, a suspension, an elixir, an emulsion, a liniment, an ointment, a plaster, a poultice, a transdermal absorption formulation, a lotion, an inhalant, an aerosol, an injection or a suppository.

15. The pharmaceutical composition according to any one of claims 11-14, which is **characterized in that**: the pharmaceutical composition is suitable for administration by a variety of routes, including oral, intravenous, subcutaneous, intramuscular, intracerebral, intranasal, pulmonary, intra-arterial, intra-articular, intradermal, intravitreal, intraosseous infusion, intraperitoneal, intrathecal, or transdermal administration.

16. The pharmaceutical composition according to any one of claims 11-15, which is **characterized in that**: the rheumatic disease is one or more selected from rheumatoid arthritis, juvenile idiopathic arthritis, psoriasis, psoriatic arthritis, ankylosing spondylitis, undifferentiated spondyloarthropathy, systemic lupus erythematosus, systemic sclerosis, collagen disease, Crohn's disease, and ulcerative colitis, and preferably, the patient having rheumatic diseases is tuberculosis-positive or tuberculosis-negative; and the patient having rheumatic diseases is or is not complicated with hepatitis B.
